# EUROPEAN PATENT APPLICATION

(11) **EP 1 905 430 A1**
(43) Date of publication of application: **02.04.2008**
(21) Application number: 06767961.3
(22) Date of filing: 07.07.2006
(51) Int. Cl.: A61K 9/20, A61J 3/06

(54) **TABLET**

(30) Priority: 08.07.2005 JP 2005199588
(71) Applicant: Takeda Pharmaceutical Company Limited, Osaka-shi, Osaka 541-0045 (JP)
(72) Inventor: SAITO, Toshihide c/o TAKEDA PHARMACEUTICAL COMPANY, Osaka-shi, Osaka 5328686 (JP); FUKADA, Hiroshi c/o TAKEDA PHARMACEUTICAL COMPANY, Osaka-shi, Osaka 5328686 (JP); ARIMA, Hiroaki c/o TAKEDA PHARMACEUTICAL COMPANY, Osaka-shi, Osaka 5328686 (JP)
(74) Representative: Fleuchaus, Michael A.
(86) International application number: PCT/JP2006/313551
(87) International publication number: WO 2007/007659

(57) **Abstract**

The present invention can inhibit the occurrence of defective products without increasing the hardness of a tablet even if stress is applied to the tablet on transporting it or packing it by an automatic dispensing machine or when taking it out of the PTP and besides can be put into practice easily and inexpensively to result in facilitating the improvement of the productivity. A tablet according to the present invention comprises a body portion (2) and cap portions (3) projecting from an upper and a lower surfaces (4) from the body portion (2) each in a convex shape. The cap portion (3) has a peripheral edge portion (7) a surface of which is provided with an upright angle (α) of 35 to 50 degrees with respect to the upper and lower surfaces (4) of the body portion (2) and is formed in a convex curved surface which becomes arcuate in vertical section. The cap portion (3) has its surface provided with an angle (β) of at least 20 degrees with respect to the upper and lower surfaces (4) at a position (9) recessed by a 5% length (L) of a horizontal length (D) of the body portion (2) from a peripheral edge portion of the body portion (2). The peripheral edge portion (7) has its surface connected to the surface at the mid portion (6) smoothly.

## Description

### Technical Field

The present invention relates to a tablet and more particularly, a tablet which can inhibit the occurrence of the defect without increasing its consistency even if it undergoes a stress while it is being transported and packed by an automatic dispensing machine or when it is taken out of the PTP and besides can be put into practice easily at a low cost and facilitate the improvement of the productivity.

### Background Art

A tablet which disintegrates in an oral cavity for a short time, so-called an oral disintegrating tablet is made by pressurizing pharmaceutical ingredients by a tabletting machine and pressing to solidify the thus pressurized pharmaceutical ingredients into a shape comprising a body portion of a predetermined thickness and cap portions formed by projecting an upper and a lower surfaces of the body portion each in a convex shape as well as conventional tablets (for example, see Patent Literature 1). At this time, for example, as shown in Fig. 6, the cap portion 51 has a peripheral edge portion 52 a surface of which is formed into a straight line smoothly inclined in vertical section with respect to the upper and lower surfaces 54 of the body portion 53 with an angle of inclination (α) (hereafter, referred to as an "upright angle") being set to about 30 degrees. Further, the cap portion 51 has at its mid portion 55 a height (hereafter, referred to as "depth (H)") usually set to less than 0.5 mm although it differs depending on the size of the tablet.

The above-mentioned tabletting increases the consistency of the tablet as the tabletting pressure becomes higher so that the tablet can reduce the likelihood of being broken or cracked while it is being transported. However, as for the tablet of the oral-disintegrable type, it must be set so as to have a suitable consistency for expediting the disintegration in the oral cavity and therefore cannot be excessively hardened. For this reason, it is necessary to take an appropriate measure such as disposing a damping member into an inner surface of the transporting way or decreasing the transporting speed. This makes the maintenance of the whole tablet producing apparatus troublesome and impossible to be taken at a low cost to result in having entailed a problem that the productivity is not easily improved. There was another problem that for example, when dispensing the tablet by the automatic dispensing machine or taking the tablet out of the PTP, the pushing force was likely to cause some defects in the tablet, which were not easily inhibited from occurring.

Patent Literature 1: Patent Application Laid-Open No. 2000-95674

### Disclosure of the Invention

### The Problem the Invention Intends to Solve

The present invention has a technical object to solve the above-mentioned problems and provide a tablet which can inhibit the occurrence of the defects without increasing its consistency even if it undergoes a stress while it is being transported and packed by an automatic dispensing machine or when it is taken out of the PTP and besides can be put into practice easily at a low cost and facilitate the improvement of the productivity.

### Means for Solving the Problem

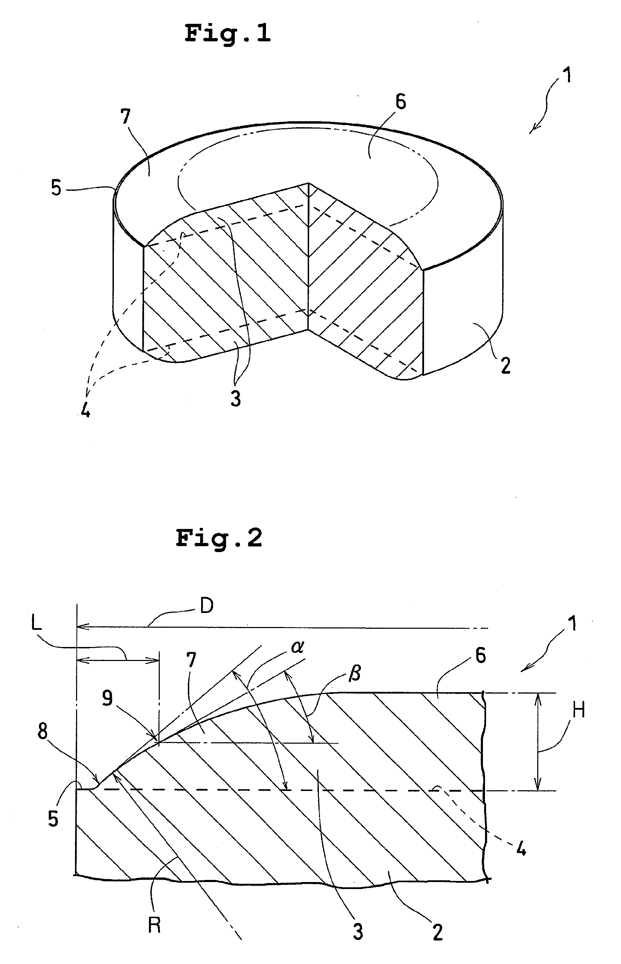

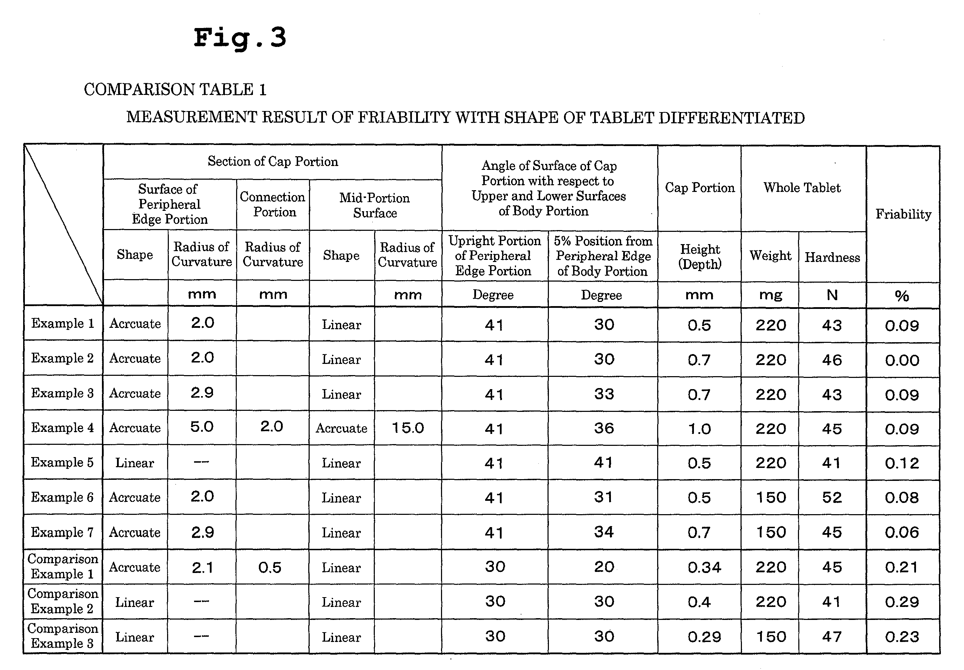

The present invention is constructed as follows so as to accomplish the above-mentioned object, for example, if it is explained based on Figs. 1 to 5 which show an embodiment of the present invention.
More specifically, the present invention concerns a tablet, which comprises a body portion 2 of a predetermined thickness and a cap portion 3 formed by projecting at least one of an upper and a lower surfaces 4 of the body portion 2 in a convex shape. The cap portion 3 has a peripheral edge portion 7 a surface of which has an upright angle (α) of 35 to 50 degrees with respect to the upper and lower surfaces 4 of the body portion 2.

Here, it is sufficient if the surface of the peripheral edge portion has an upright angle of substantially 35 to 50 degrees, preferably 35 to 45 degrees. Although the surface of the peripheral edge portion may be raised by this upright angle directly from the upper and lower surfaces, the upright portion with this upright angle has a surface connected to a peripheral edge portion (so-called a land portion) outside the cap portion of the upper and lower surfaces of the body portion by a smoothly curved surface.

The surface of the peripheral edge portion may be linear in vertical section. However, in the event that the surface is formed in a convex curved shape comprising one or plural arcs in vertical section, even if the upright angle is made larger, it is easy to connect the surface to the mid portion of the cap portion smoothly. So this is preferable. In this case, although the convex curved surface is not limited to have a specific radius of curvature, the surface of the peripheral edge portion has an angle with respect to the upper and lower surfaces of the body portion, preferably set to be large over a predetermined range including the upright portion with the upright angle. For example, it is preferably set to at least 20 degrees, more preferably 20 to 90 degrees and most preferably 20 to 45 degrees at a position where the surface is recessed by 5% of a horizontal length of the body portion from the peripheral edge of the body portion.

Although the cap portion may have at a mid portion a surface formed from a convex curved surface, if at least most part thereof is made planar in parallel to the upper and lower surfaces of the body portion, the tablets do not superpose one on another and therefore are arranged evenly at a predetermined position while they are being transported. So this is preferable. However, the cap portion may have its surface provided with a dividing line for easy division, letters or numerals for identification and symbols by debossing or with these letters by printing. Further, here the above-mentioned terms "most part" may be wide enough for the tablet to retain the stable position. Concretely speaking, it means at least about 20% of the projected area of the whole tablet in plan view.

The cap portion may be formed by connecting the surface of the peripheral edge portion to the surface of the mid portion through an angular portion of a large intersection angle. However, preferably, they are connected smoothly to each other because even if they experience a stress, they are prohibited from breaking at this connection portion.

As for the height at the mid portion of the cap portion, namely the degree of depth, an excessive enlargement causes the cap portion to be separated from the body portion to produce a defective tablet. On the other hand, an excessive decrease reduces the range having a large angle, which includes the upright angle, with respect to the upper and lower surfaces of the body portion. For this reason, the cap portion preferably has a degree of depth of about 0.3 to 1.5 mm.

The above-mentioned tablet may be applied to the conventional tablet. However, when it is applied particularly to the so-called oral disintegrating tablet, the body portion and the cap portion of which disintegrate in an oral cavity for a short time, it can remarkably exert the effect of the invention, that is to say, "the possibility of inhibiting the occurrence of defects without increasing the consistency of the tablet upon application of the external stress" to be mentioned later and therefore, this is preferable.

The tablet of the present invention is suitably applied to the pharmaceutical medicine including an optional one or plural kinds of pharmaceutical active ingredients.
Here listed as examples of the above-mentioned pharmaceutical active ingredients are, for example, α-glucosidase inhibitors such as voglibose, acarbose, miglitol and emiglitate; lansoprazole, omeprazole, rabeprazole, pantoprazole, ilaprazole, tenatoprazole or their proton pump inhibitors; insulin sensitizers such as pioglytazone hydrochloride, troglitazone and rosiglitazone; and angiotensin-II receptor antagonists such as losartan, eprosartan, candesartan, valsartan, telmisartan, irbesartan, olmesartan, tasosartan and candesartan cilexetil.

The tablet of the present invention, for example the oral disintegrating tablet can be formulated into a dosage form suitable for oral administration by using, for example, water-soluble sugar alcohol, crystalline cellulose, low-substituted hydroxypropylcellulose in addition to the pharmaceutical active ingredient and further adding and mixing a binder, a sour agent (acid), a foaming agent, an artificial sweetening agent, a flavorant, a lubricant, a coloring agent, a stabilizer, an excipient and a disintegrant and then subjecting them to a compression-molding process according to a method known per se. Further, the tablet can be formulated into a molded tablet by separately pouring aqueous solution of a pharmaceutical active ingredient dispersed therein, into a mold, for example, such as pockets for molding PTP and drying it by a freeze-dryer or an air-flow dryer and thereafter heat-sealing it.

The above-mentioned "water-soluble sugar alcohol" means a sugar alcohol which requires less than 30 ml of water when 1g of sugar alcohol is added to the water and shaking and mixing them for 30 secs. every 5 mins. at a temperature of 20 degrees C to solve it within about 30 mins.
Examples of the above-mentioned "water-soluble sugar alcohol" are, for example, mannitol, sorbitol, maltritol, reduced starch diastatic substance, xylitol, reduced parachinose and erythritol. At least two of them may be mixed at a suitable ratio.
Mannitol, xylitol and erythritol are preferable for the water-soluble sugar alcohol. Ordinarily, about 3 to 50 parts by wt, preferably about 5 to 30 parts by wt of the water-soluble sugar alcohol is used for 100 parts by wt of the whole pharmaceutical medicine.

As for the crystalline cellulose, it may be made by partially depolymerize α-cellulose and purifying the thus depolymerized one. Further, it also contains the cellulose called fine-crystalline cellulose. Concrete examples of this crystalline cellulose are CEOLUS KG 801, CEOLUS KG 802, avicel PH 101, avicel PH 102, avicel PH 302 and avicel RC-591(crystalline cellulose carmellose sodium). More preferred examples of the crystalline cellulose are CEOLUS KG 801 or CEOLUS KG 802 which is called avicel of high compressibility. As for these crystalline celluloses, one species may be used singly or alternatively at least two species may be use in combination. These crystalline celluloses are commercially available (for example the products manufactured by Asahi Chemical Co., Ltd.).
As for the crystalline cellulose, it may be blended in the amount of about 3 to 50 parts by wt, preferably about 5 to 40 parts by wt and most preferably about 5 to 20 parts by wt in proportion to 100 parts by wt of the whole pharmaceutical medicine.

The above-mentioned "low-substituted hydroxypropylcellulose" means low-substituted hydroxypropylcellulose, in which hydroxypropoxyl-group content (hereafter it may be some times abbreviated as HPC-group content) in hydroxypropylcellulose is about 0.5 to 9.9. wt%, among others, low-substituted hydroxypropylcellulose of about 5.0 to 7.0 wt % hydroxyl-group content and low-substituted hydroxypropylcellulose of about 7.0 to 9.9 wt % hydroxyl-group content.
Listed as examples of the low-substituted hydroxypropylcellulose of about 7.0 to 9.9 wt % HPC-group content are LH-22, LH-32 and a mixture of them. These are commercially available (as the product manufactured by Shin-Etsu Chemical Co., Ltd.) Listed as examples of the low-substituted hydroxypropylcellulose of about 5.0 to 7.0 wt % HPC-group content are LH-23, LH-33 and a mixture of them.

The low-substituted hydroxypropylcellulose of about 5.0 to 7.0 wt % hydroxypropyl-group content has a particle diameter of, for example, about 5 to 60 µm, preferably about 10 to 40 µm as an average particle diameter.
In the case where L-HPC of a larger particle diameter within such a range (for example, L-HPC having an average particle diameter of about 26-40 µm) is used, it is possible to make pharmaceutical medicine in excoreent disintegrability. On the other hand, in the event that L-HPC of a smaller particle diameter within such a range (for example, L-HPC having an average particle diameter of about 10 to 25 µm) is used, it is possible to make pharmaceutical medicine of excoreent hardness. In consequence, the particle diameter of L-HPC can be appropriately selected depending on the characteristics of the intended pharmaceutical medicine.
As for the low-substituted hydroxypropylcellulose, it may be blended in the amount of generally about 1 to 50 parts by wt, preferably about 1 to 40 parts by wt and more preferably about 5 to 20 parts by wt in proportion to 100 parts by wt of the whole pharmaceutical medicine in order to obtain sufficient oral-disintegrability and hardness for the pharmaceutical medicine.

Examples of the binder are, for example, hydroxypropylcellulose, hydrokypropylmethylcellulose, crystalline cellulose, α -starch, polyvinylpyrrolidone, gum arabic, gelatin, pullulan and so on. As for these binders, at least two pieces may be mixed at a suitable ratio for use. In the case of using the crystalline cellulose as the binder, it is possible to obtain solid pharmaceutical medicine of larger hardness while retaining excoreent oral-disintegrability.
Here the crystalline cellulose may be the cellulose manufactured by partially depolymerizing the α -cellulose and purifying it. Further, it also contains a cellulose called fine crystalline cellulose. Concrete examples of this crystalline cellulose are CEOLUS KG 801, CEOLUS KG 802, avicel PH 101, avicel PH 102, avicel PH 301, avicel PH 302, avicel RC-A591NF(crystalline cellulose carmellose sodium) and avicel RC-A591 (crystalline cellulose carmellose sodium). Among others, CEOLUS KG 801 or CEOLUS KG 802 which is called avicel of high compressibility is suitably used. As for these crystalline celluloses, at least two species may be mixed at an appropriate ratio for use. Besides, these crystalline celluloses are commercially available (for example the products manufactured by Asahi Chemical Co., Ltd.)
In the case where the crystalline cellulose is a solid pharmaceutical medicine which includes no fine particle, for example, 1 to 50 parts by wt, preferably 2 to 40 parts by wt, more preferably 2 to 20 parts by wt of the crystalline cellulose is used for 100 parts by wt of the whole pharmaceutical medicine.

Examples of the sour agent (acid) are, for example, citric acid (citric anhydride), tartaric acid and malic acid.
Examples of the foaming agent is sodium bicarbonate.
Examples of the artificial sweetening agent are saccharin sodium, dipotassium glycyrrhizinate, aspartame, stevia, thaumatin and so on.
Flavorant may be either synthetic or natural and its examples are lemon, lemon lime, orange, menthol, strawberry and so on
Examples of the lubricant are magnesium stearate, cane sugar-fatty acid ester, polyethylene glycol, talc and stearic acid. With the polyethyleneglycol used as the lubricant, it is possible to obtain a stable solid pharmaceutical medicine which has suppressed the decomposition of its pharmaceutical ingredient with the elapse of days. In this case, for example, 0.01 to 10 parts by wt, preferably 0.1 to 5 parts by wt of polyethyleneglycol is used for 100 parts by wt of the whole pharmaceutical medicine.
Examples of the coloring agent are FD & C Yellow No. 5, FD & C Blue No. 2 and the like food colorants; food lakes, red iron oxide and so on.
As for the stabilizer, when it is a pharmaceutical ingredient of base-group, its examples are basic substances. On the other hand, if it is a pharmaceutical ingredient of acid-group, its examples are acid substances.
Examples of the excipient are lactose, sucrose, D-mannitol (β-D-mannitol), starch, corn starch, crystalline cellulose, light anhydrous silicic acid, titanium oxide and so on.

Examples of the disintegrant are a disintegrant agent, which is called "super disintegrant), such as crospovidone (for example, manufactured by ISP Inc. (U.S.A) or BASE Company (Germany)), croscarmellose sodium (for example, made by FMC-Asahi Chemical Co., Ltd.), carmellose calcium (for example, made by Gotoku Chemical (Yakuhin)); hydroxypropylcellulose, low-substituted hydroxypropylcellulose; carboxymethylstarch sodium (for example, made by Matsutani Chemical Co., Ltd.); corn starch. Among others, crospovidone is suitably used. As for these disintegrants, at least two species may be mixed at an appropriate ratio for use.

As for the crospovidone, any cross-linked homopolymer which is called 1-ethenyl-2-pyrrolidinonehomopolymer and includes those called polyvinylpyrrolidone (PVPP) and 1-vinyl-2-pyrrolidinonehomopolymer is sufficient. Ordinarily, crospovidon of at least 1,000,000 molecular weight is employed. Concrete examples of the commercially available crospovidone are, for example, cross-liked povidone, corrydone CL (made by BASF Co., Ltd. (Germany)), polyplusdone XL, polyplusdone XL-10, INF-10 (made by ISP Inc. (U.S.A.), polyvinylpolypyrrolidone, PVPP, 1-vinyl-2-pyrrolidinonehomopolymer.
As for these disintegrants, only one piece may be used, or alternatively at least two pieces may be used in combination. For example, crospovidone may be used alone or in combination with another disintegrant.
As regards such a disintegrant, it is used in the amount of, for example, 0.1 to 20 parts by wt, preferably 1 to 10 parts by wt and more preferably 3 to 7 parts by wt in proportion to 100 parts by wt of the whole pharmaceutical medicine.

In the case where the pharmaceutical active ingredients are unstable to acids such as lansoprasole, omeprasole, labeprasole and pantprasole, it is preferable to blend a basic inorganic salt so as to stabilize this pharmaceutical active ingredient in the pharmaceutical medicine. Examples of this basic inorganic salt are basic inorganic salts of sodium, potassium, magnesium and/or calcium. Basic inorganic salts of magnesium and/or potassium are preferable. Among others, basic inorganic salt of magnesium is more preferable.
Examples of the basic inorganic salt of sodium are, for example, sodium carbonate, sodium bicarbonate, sodium phosphate, sodium dihydrogen phosphate.
Examples of the basic inorganic salt of potassium are, for example, potassium carbonate, potassium bicarbonate, sodium potassium carbonate, potassium phosphate, potassium dihydrogenphophate.
Examples of the basic inorganic salt of magnesium are, for example, heavy magnesium carbonate, magnesium carbonate, magnesium oxide, magnesium hydroxide, magnesium aluminate metasilicate, magnesium aluminate silicate, magnesium silicate, magnesium aluminate, hydrotalsite [Mg₆Al(OH)₁₆ • CO₃ • 4H₂O] and alumina • magnesium hydroxide[2.5MgO • Al₂O₃ • ×H₂O]. Among others, heavy magnesium carbonate, magnesium carbonate, magnesium oxide and magnesium hydroxide are preferable.
Examples of the basic inorganic salt of potassium are, for example, precipitated potassium carbonate and potassium hydroxide.

The basic inorganic salt is basic inorganic salt of preferably magnesium, and more preferably heavy magnesium carbonate, magnesium carbonate, magnesium oxide and magnesium hydroxide. As regards each of these basic inorganic salts of magnesium and potassium and the like, it is satisfactory if its 1% aqueous solution or suspension has pH indicating that it is basic (pH of at least 7).
As for each of these basic inorganic salts (preferably salts of magnesium and of potassium), at least two pieces may be blended at a suitable ratio for use.
As regards the amount of the basic inorganic salt to be used, it may be appropriately selected depending on the kind of the basic inorganic salt. For example, it is 0.3 to 200 wt%, preferably 1 to 100 wt%, more preferably 10 to 50 wt% and most preferably 20 to 40 wt% in proportion to the pharmaceutical ingredient.

The oral disintegrating tablet according to the present invention may contain fine particles, which may include a pharmaceutical active ingredient. This tablet can be manufactured by a publicly known method or a method similar thereto.
The fine particles may contain a core including or excluding a pharmaceutical active ingredient. Examples of such core are a spherical product granulated from crystalline cellulose and lactose and a spherical product granulated from crystalline cellulose. Examples of the crystalline cellulose-lactose spherical granulated product are a spherical product granulated from 3 parts of crystalline cellulose and 7 parts of lactose and having a diameter of about 100 to 200 µm (for example, made by Freund Corporation.; Trade Name of NONPAREIL 105), a spherical product granulated from 3 parts of crystalline cellulose and 7 parts of lactose and having a diameter of about 150 to 250 µm (for example, made by Freund Corporation; Trade Name of NONPAREIL NP-7:3) and a spherical product granulated from 5 parts of crystalline cellulose and 5 parts of lactose and having a diameter of about 150 to 250 µ m (for example, made by Freund Corporation.; Trade Name of NONPAREIL-5:5). An example of the spherical product granulated from crystalline cellulose is, for example, a spherical product granulated from crystalline cellulose having a diameter of about 150 µ m to 250 µ m (for example, made by Asahi Chemical Co., Ltd.; Trade Name of AVICEL SP).

The above-mentioned core is coated with a pharmaceutical active ingredient and then may be coated by a method known per se so as to mask a smell or taste for enteric or controlled-release purpose. At this time, the core forms fine particles containing the pharmaceutical active ingredient.
In the above case, examples of the coating agent are an aqueous enteric macromolecular base agent such as celluloseacetatephtarate (CAP), hydroxypropylmethylcellulosephtharate (hereafter may be referred to as HP-55), hydroxymethylcellulose acetate succinate, copolymer methaclyrate (for example, made by Rohm ; Trade Name Eudragit L30D-55, made by BASF Company; Trade Name of COLICOAT MAE30DP, made by Sanyo Chemical Industrial Co., Ltd.; Trade Name of POLIKID PA30), carboxymethylethylcellulosse, ceramic and so on, which is used for forming an enteric coating layer; a base agent for controlling the release such as copolymer methaclyrate (for example, made by Rohm; Trade Name Eudragit NE30D, Eudragit RL30D and Eudragit RS30D) and a plasticizer such as water-soluble macromolecule, triethyl citrate, polyethyleneglycol, monoglyceride acetilate, triacetine, caster oil and so on. One or at least two of them may be mixed for use.

The aforesaid enteric coating is preferably formed in combination with an aqueous enteric macromolecular base agent and a base agent for controlled-release and where necessary, with a plasticizer.
Copolymer methacrilate is preferable for the aqueous enteric macromolecular base agent.
Copolymer methacrilate is preferable a base agent for the controlled-release.
The release-controlling base agent is used in the amount of about 5 to 30 wt%, preferably about 5 to 15 wt% in proportion to 100 wt% of the aqueous enteric macromolecular base agent. The plasticizer is preferably used in the amount of 5 to 30 wt% in proportion to 100 wt% of the aqueous enteric macromolecular base agent.

The above-mentioned "fine particles" can be produced by a publicly known granulating method.
Examples of the "granulating method" are the agglomeration granulating method (for example, centrifugal fluidized-bed granulating method), the flow granulating method (for example, rotating fluidized-bed granulation, flow granulation) and the agitating granulation method. Among others, the flow granulating method is preferable and the rotating fluidized-bed granulation method is particularly preferable.
A concrete example of this agglomeration granulating method is, for example, a method using "CF Apparatus" made by Freund Corporation. Listed as concrete examples of the rotating fluidized-bed granulation method are, for example, the method using "SPIRAFLOW", "MULTIPLEX" manufactured by Powrex Corporation and "NEWMALME" by Fuji Powdal Co., Ltd. The way to spray the mixture solution can be chosen appropriately depending on the kinds of the granulating apparatus. It may be any one of the top-spray system, bottom-spray system and tangential-spray system. Among others, the tangential-spray system is preferable.

The "fine particles" can be coated with ingredients other than fine particles including a pharmaceutical ingredient or the like by a method known per se or a method similar thereto. For example, in the case where the pharmaceutical active ingredients are physiologically active substances unstable to acid, there is used a method to coat the core, which contains crystalline cellulose and lactose, with a physiologically active substance unstable to acid.

An example of the production method (coating method) disclosed in Japanese Patent Application Laid-Open No. 5-092918 is a method for coating the core, which includes crystalline cellulose and lactose, with a physiologically active substance unstable to acid and where necessary, a basic inorganic salt, a binder, a lubricant, an excipient, a water-soluble polymer (hereafter, some times, abbreviated as "coating layer"). Listed as an example is a method to coat the core with a physiologically active substance unstable to acid and a basic inorganic salt and besides with a binder, a lubricant, an excipient and a water-soluble polymer.

As for the basic inorganic salt, binder, lubricant and excipient, the above-mentioned ones are used. Further, as for the "water-soluble polymer", for example, water-soluble polymer which can or cannot be solved in ethanol is used. Examples of the water-soluble polymer, which can be solved in ethanol, are, for example, a cellulose derivative of hydroxypropylcellulose (hereafter may be referred to as "HPC"), polyvinylpyrrolidone and so on. Examples of the water-soluble polymer, which cannot be solved in ethanol, hydroxypropylmethylcellulose (hereafter, some times referred to as "HPMC"), methylcellulose, cellulose derivative of sodium carboxymethyl cellulose, sodium polyacrilate, polyvinyl alcohol, sodium alginate, guar gum and so on.

The mean particle diameter of the above-mentioned "core" is sufficient if it is not more than 250 µm, preferably 50 to 250 µm, more preferably 100 to 250 µm, particularly preferably 100 to 200 µm. The core of such a mean particle diameter includes the particles that have fully passed through a sieve of No. 50 (300 µ m) and remained at a sieve of No. 60 (250 µm) and occupy about not more than 5w/w% of the total amount and the particles that have passed through a sieve of No. 282 (53 µm) and occupy not more than about 10 w/w% of the total amount. The specific volume of the "core" is not more than 5 ml/g and preferably not more than 3 ml/g.

Examples of the core are, for example, (1) a spherical product granulated from crystalline cellulose and lactose; (2) a spherical product of 150 to 250 µ m granulated from crystalline cellulose (for example made by Asahi Chemical Co., Ltd.; Trade Name; AVICEL SP); (3) a product of 50 to 250 µm agitation-granulated from 9 parts of lactose and 1 part of starch, (4) fine particles obtained by classifying spherical granules of fine crystalline cellulose which is disclosed in the publication of Japanese Patent Laid-Open No. 61-213201 and having a diameter of not more than 250 µm, (5) processed product such as waxes spherically formed by spraying-chilling or molt-granulation, (6) processed product such as gelatin micro beads of oil ingredient, (7) calcium silicate, (8) starch, (9) porous particles of chitin, cellulose, chitosan and so on, (10) bulk product and its pharmaceutically processed product such as granulated sugar, crystalline lactose, crystalline cellulose or sodium chloride. Further, these cores may be produced by a crushing method known per se or a granulation method and then passed through a sieve so as to prepare particles of a desired diameter.

Examples of the spherical product granulated from crystalline cellulose and lactose are, for example, (1) a spherical product granulated from 3 parts of crystalline cellulose and 7 parts of lactose and having a diameter of 100 to 200 µm (for example, made by Freund Corporation; Trade Name of NONPAREIL 105 (70-140), particle diameter of 100 to 200 µm); (2) a spherical product granulated from 3 parts of crystalline cellulose and 7 parts of lactose and having a diameter of 150 to 250 µm (for example, made by Freund Corporation; Trade Name of NONPAREIL NP-7:3; (3) a spherical product granulated from 4.5 parts of crystalline cellulose and 5.5 parts of lactose and having a diameter of 100 to 200 µm (for example, made by Freund Corporation ; Trade Name of NONPAREIL 105T (70-140), particle diameter of 100 to 200 µm); (4) a spherical product granulated from 5 parts of crystalline cellulose and 5 parts of lactose and having a diameter of 150 to 250 µm (for example, made by Freund Corporation; Trade Name of NONPAREIL NP-5:5).

Examples of the core used in order to produce pharmaceutical medicine excellent in solubility while retaining a suitable hardness are preferably a spherical product granulated from crystalline cellulose and lactose and more preferably a spherical product granulated from crystalline cellulose and lactose and containing 50 wt% of lactose. Preferable spherical granulated product contains 40 to 50 wt% of crystalline cellulose and 50 to 60 wt% of lactose.
The core to be used in the present invention is preferably a spherical product granulated from crystalline cellulose and lactose and more preferably a spherical product granulated from 4.5 parts of crystalline cellulose and 5.5 parts of lactose and having a diameter of 100 to 200 µm.

The core may include physiologically active substance such as the above-mentioned pharmaceutical active ingredient. However, a coating layer including this physiologically active substance can control the release of the physiologically active substance and therefore the core may not include the physiologically active substance.
Here the core may be of fine particles and preferably of a sphere as uniform as possible so as to reduce the unevenness of the coating.

The ratio of the coating layer to the core can be selected within such a range as being able to control the solubility of the physiologically active substance and the grading of the composition. For example, it is ordinarily 50 to 40 parts by wt with respect to 100 parts by wt of the core.
The above-mentioned "coating layer" may be formed from a plurality of layers. And it is sufficient if at least one of the plural coating layers contains a physiologically active substance. It is possible to appropriately select a combination of various coating layers such as a coating layer or a ground coating layer which does not include any pharmaceutical active ingredient, an enteric coating layer or the like.

In the case of coating the aforesaid core, for example, the foregoing physiologically active substance and water-soluble polymer are used as mixture solution. This mixture solution may be solution or suspension and can be prepared by using an organic solvent such as water or ethanol or a mixture thereof.
The concentration of the water-soluble polymer in the mixture solution is usually 0.1 to 50 wt% and preferably 0.5 to 10 wt%, although it may differ depending on the physiologically active substance and the other additives, in order to retain the binding force of the physiologically active substance with respect to the core and to keep the viscosity of the mixture solution to such a degree as not to reduce the workability.

In the event that the coating layer is formed from a plurality of layers, the layers are coated one on another by choosing the blending ratio of the water-soluble polymer and the grading of the viscosity and using the mixture solution which has the ratios of physiologically active substance and other additives varied. And the physiologically active substance contained in each of the layers may vary its concentration continuously and gradually. In this case, as far as the whole coating layer contains 0.1 to 50 wt% of the water-soluble polymer, it may be coated with mixture solution of blending ratio outside the range of 0.1 to 50 wt%. Additionally, an inactive coating is formed by a method known per se and a plurality of the thus formed inactive coating may constitute a multi-layer coating so that it can shield a space between every pair of adjacent layers containing the physiologically active substance.
Moreover, in the case where at least two physiologically active substances of bad blending characteristic are combined, the respective mixture solution may be used simultaneously or separately so as to coat the cores.

After the coated substance has been dried, it is passed through a sieve to obtain a composition of even grading. The composition generally has a shape corresponding to the core. Therefore, it is possible to obtain the composition of approximately sphere. Usable for the sieve is, for example, a round sieve of No. 50 (300 µm). A composition is attained by selecting that which has passed through this sieve of No. 50.

The fine particles are produced by coating the composition with an enteric coating layer for the purpose of protecting the physiologically active substance or applying the enteric characteristic in accordance with the same granulation method as mentioned above. If required, it may be further coated with water-soluble sugar alcohol (preferably mannitol). If it is coated with water-soluble sugar alcohol, it may improve the hardness of the oral disintegrating tablet which contains fine particles.
As a preferable example of the enteric coating layer, for example, an enteric macromolecular base agent, a base agent for controlled release and a plasticizer such as the above-mentioned ones are combined together to prepare a coating layer, which coats an entire surface of a composition including the aforesaid pharmaceutically active ingredient in a thickness of 20 to 70 µm, preferably 30 to 50 µm. Accordingly, as this composition has particles of a smaller diameter, the enteric coating layer has a larger wt% which occupies the total amount of the fine particles. The enteric coating layer is 30 to 70 wt % and preferably 50 to 70 wt % of the total amount of the fine particles.
The enteric coating layer may consist of plural, for example, 2 to 3 layers. For example, there is listed a coating method which comprises coating a composition with an enteric coating layer including polyethyleneglycol, with another enteric coating layer including triethyl citrate and with still another enteric coating layer including polyethyleneglycol.

The oral disintegrating tablet is produced by a conventional method of producing the pharmaceutical medicine. An example of such a method comprises: mixing a pharmaceutically active ingredient, for example, mannitol or the like water-soluble sugar alcohol and where necessary, crystalline cellulose and/or low-substituted hydroxypropylcellulose and other additives; contacting the mixture with aqueous solvent of a predetermined amount (for example, water, methanol, ethanol and the like lower alcohol; acetone; or mixture solvent thereof; preferably water); and then drying it. As to the molding, it may be effected by a conventional method before or after drying, alternatively while drying it.

The oral disintegrating tablet including fine particles is appropriately produced by a conventional molding method with a suitable active ingredient selected from the above-mentioned group of physiologically active substances.
For example, an oral disintegrating tablet including fine particles can be produced by mixing fine particles including a pharmaceutically active ingredient with various sorts of additives and tabletting the thus resulting mixture.
Examples of the fine particles including pharmaceutically active ingredient are the above-mentioned fine particles each including a coated core and the like.

An example of a method for producing the oral disintegrating tablet by using the additives and the fine particle including a coated core is recited below.
The used additives may contain water-soluble sugar alcohol such as mannitol, crystalline cellulose and/or low-substituted hydorxypropylcellulose and where necessary, various sorts of additives exemplified as above such as a binder, a sour agent (acid), an artificial sweetening agent, a flavorant, a lubricant, a coloring agent, a stabilizer and an excipient and a disintegrant.

As for the crystalline cellulose, it may be blended in the ratio of about 3 to 50 parts by wt, preferably about 5 to 40 parts by wt and most preferably about 5 to 20 parts by wt, in proportion to 100 parts by wt of the ingredients other than the fine particles of the oral disintegrating tablet.
Further, as regards the low-substituted hydroxypropylcellulose, for example, low-substituted hydroxypropylcellulose containing 5.0 to 7.0 wt% of HPC base or low-substituted hydroxypropyl cellulose containing 7.0 to 9.90 wt% of HPC base, it is ordinarily used in the amount of about 3 to 50 parts by wt, preferably about 5 to 40 parts by wt and more preferably 5 to 20 parts by wt in proportion to 100 parts by wt of the ingredients other than the fine particles of the oral disintegrating tablet in order to obtain a sufficient oral-disintegrability and hardness for the pharmaceutical medicine.
Although a mixture of the various sorts of additives as mentioned above may be used in the state of powder as it is for the additive containing water-soluble sugar alcohol, it may be granulated by a conventional granulating method.

The granulating means is not particularly limited, but the dry granulating method known per se, the wet granulating method such as the extrusion granulation, fluidized bed granulation and agglomeration granulation, and the spraying method are usable.
The dry granulating method comprises using raw powder material as it is or mixing it with a suitable binder and applying strong pressure to form a small mass and then appropriately crushing the small mass to granulate it.
The wet granulating method comprises adding to the raw powder material, solution or suspension of the suitable binder mentioned above and mixing them together and then granulating, drying and uniformizing the mixture. The wet raw powder material may be vibrated or rotated and then rolled so as to be formed into minute spherical particles.
The spraying method includes a step of spraying slurry raw material through a nozzle or a rotary disk in the state of extremely small liquid drops and blowing hot air thereto for drying them.

Here the extrusion granulation means a method which comprises adding a solvent to a mixture of a pharmaceutical ingredient and additives or a pharmaceutical ingredient alone to mix and kneed them, applying pressure by a screw, roller and so on to the thus kneaded mixture and then extruding the mixture through a screen or a die.
The fluidized-bed granulating method means a method of spraying a solution containing a binder to a mixture of a pharmaceutical ingredient and additives or a pharmaceutical ingredient alone while retaining it in a flow state, and converge the powder materials to each other by the binder for granulation.
The agglomeration-granulation means a method of forming cross-linking between particles to generate fine particles by rolling them within a container of any one of pan-type, drum-type and vibration-type through the action of agitating blades while spraying an aqueous solution containing a binder and applying a rolling and rotating movement to the fine particles so as to improve their growth.

In the case of granulating the additive containing water-soluble sugar alcohol such as mannitol, the wet granulating method such as the fluidized-bed granulating method is particularly preferable.
For example, mannitol and where necessary, crystalline cellulose and/or low-substituted hydroxypropylcellulose and other additives (for example, the binder, sour agent (acid), foaming agent, artificial sweetening agent, flavorant, lubricant, coloring agent, stabilizer, excipient and disintegrant) are inserted as solid ingredients into a fluidized-bed granulating machine and solution (preferably aqueous solution) or suspension of mannitol made by aqueous solvent (for example, water, methanol, ethanol and the like lower alcohol; acetone; or mixture solvent of them) is sprayed and dried so as to be able to obtain dry powder The fluidized-bed granulation includes the step of spraying the solution including mannitol or the like to the composition of additives inserted as a solid ingredient and the step of drying them.

After dry powder of additives has been produced by the fluidized-bed granulating method, the obtained dry powder of additives may be used to the next step or be uniformized.

As for the fine particles including the coated core, more concretely, for example, a core-including crystalline cellulose and lactose is coated with a physiologically active substance and an excipient and further with a coating layer which includes a water-soluble polymer to obtain a composition. The thus obtained composition is coated successively with an enteric coating layer containing polyethyleneglycol, another enteric coating layer containing triethyl citrate, still another enteric coating layer containing polyethyleneglycol and with mannitol to attain fine particles. The attained fine particles are mixed with an additive agent containing water-soluble sugar alcohol of mannitol or the like and the resulting mixture is molded. This method is listed as an example.

The "molding" is effected by using the single tabletting machine (manufactured by Kikusui Seisakusho Co., Ltd.), the rotary tabletting machine (manufactured by Kikusui Seisakusho Co., Ltd.) and the like and tabletting under pressure of 0.5 to 3ton/cm² and preferably, 1 to 2ton/cm²
The "drying" is effected by any one of the methods to be used for the vacuum-drying, fluidized-bed drying and other drying methods generally used for making pharmaceutical medicines.
The "mixing" is effected by a generally used method of, for example, mixing, kneading and granulating. This mixing is transported out by, for example, the vertical granulator VG10 (manufactured by Powrex Corporation), the universal kneading machine (manufactured by Hata Iron Works Co., Ltd.), the fluidized-bed granulating machine LAB-1, FD-3S (manufactured by Powrex Corporation), the coating granulation machine of rolling-flowing type MP-10, MP-400 (manufactured by Powrex Corporation) and the like device.

The oral disintegrating tablet can be made by mixing the additive obtained by granulating mannitol and the like, fine particles including the coated core and if necessary, the other additives, and tabletting the mixture.
The tabletting method is not particularly limited, but the tabletting can be performed according to the method recited in, for example, Japanese Patent Application Laid-Open No. 2003-081814 preferably at a temperature of about 25 degrees C to about 60 degrees C.

In the present specification, the term "coating" is used to mean not only the case of coating the whole surface or part of an object (for example, core) to be coated but also the case of allowing the object to adsorb or absorb.
The word "spherical" is used to mean not only "completely spherical" but also "spherical shape" having a curved sectional surface in such a shape as ellipse, eggplant and liquid drop.
The language "mean particle diameter" indicates median diameter in reference to volume (median diameter: particle diameter corresponding to accumulated distribution of 50%) as far as it is not particularly defined. An example of its measuring method is a method for measuring the grading distribution of laser-diffraction system. A concrete example is the grading-distribution measuring device of laser diffraction (HEROS RODOS (manufactured by Sympatec Co., Ltd. (Germany)).

Each of the fine particles has an average diameter of preferably not more than 400 µm and more preferably 300 to 400 µm so as not to be felt that there is something rough or wrong in the oral cavity.
In the case of defining not the "mean particle diameter" but the "largest size" of each of the "fine particles", the particle diameter is substantially not more than 425 µnm and more preferably not more than 400 µm. As for a preferable range, the particle diameter is substantially 300 to 425 µm and more preferably substantially 300 to 400 µm. Here the term "substantial" size of the particle diameter means that it may include a small amount (not more than 5 wt%) of particles each of which has a diameter outside the above-mentioned range so far as they are contaminated inevitably.

The fine particles may include titanium oxide as a masking agent.
If the oral disintegrating tablet has a diameter of 5 to 20 mm, preferably 7 to 15 mm, more preferably 8 to 13 mm, it is advantageously handled for dosage.

The oral disintegrating tablet disintegrates in oral cavity (until a solid pharmaceutical medicine completely disintegrates with saliva in an oral cavity of a healthy male or female adult) generally within 90 seconds, preferably within 1 minutes, more preferably within 5 to 40 seconds, much more preferably within 5 to 40 seconds, and particularly preferably within 5 to 35 seconds.
The oral disintegrating tablet disintegrates in water generally within 5 to 3 minutes, preferably within 5 seconds to 1 minutes, and more preferably within 5 to 35 seconds.

The tablet of the present invention, particularly the oral disintegrating tablet has generally a hardness of about 1 kg to about 15 kg (values measured by the consistency meter)
The tablet of the present invention, particularly the oral disintegrating tablet is dosed with or without water. The ways to dose it is :
(1) either including it in mouth and allowing it to be dissolved or disintegrated with salvia in oral cavity and a small or no amount of water without swallowing it;
(2) or swallowing it with water, as it is.
   Alternatively, the tablet may be dosed after it has been dissolved or disintegrated with water.
   This oral disintegrating tablet is advantageouly utilised for the cases:
   (a) where it must be mostly dosed without water;
   (b) where it is dosed by a patient who experiences a difficulty when swallowing it; and
   (c) where it is dosed by an aged person or an infant who is likely to have its throat stuffed with a tablet if the latter is a conventional one.

### Effect of the Invention

The present invention is constituted and functions as above and therefore offers the following effects.
The cap portion has a peripheral edge portion the surface of which is provided with a large upright angle. Accordingly, the tablet can be prohibited from breaking and cracking when an external stress is applied thereto without increasing its consistency.
As a result, a damping member conventionally required in the apparatus of producing tablets, can be easily attached to a transporting passage, which in turn can put the present invention into practice with ease at a low cost. Further, it makes unnecessary to take the measure for delaying the transporting speed and besides possible to reduce the occurrence of defective products when dispensing by an automatic dispensing machine and therefore easily improve the productivity. Additionally, when taking it out of the PTP, it is probable to prevent the defective products from occurring by the pushing force.

### Brief Description of the Drawings

[Fig. 1] is a partly broken perspective view of a tablet showing an embodiment of the present invention;
[Fig. 2] is a vertical sectional view of the tablet according to the embodiment of the present invention with the vicinity of its upright portion enlarged;
[Fig. 3] is a Comparison Table 1 which indicates the results of measuring the abrasion ratio with the shape of the tablet differentiated;
[Fig.4] is a Comparison Graph which indicates the results of measuring the abrasion ratio with the shape and consistency of the tablet differentiated;
[Fig. 5] is a Comparison Table 1 which indicates the results of measuring the occurrence ratio of defective products with the shape of the tablet differentiated; and

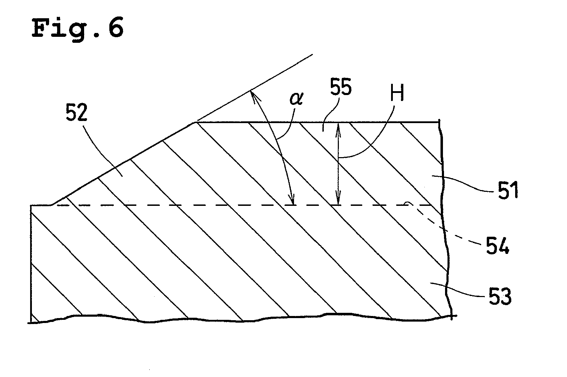

[Fig. 6] is a vertical sectional view of the tablet according to the prior art with the vicinity of its upright portion enlarged.

### Explanation of Numerals

- 1: tablet
- 2: body portion
- 3: cap portion
- 4: upper and lower surfaces of the body portion (2)
- 5: land portion
- 6: mid portion of the cap portion (3)
- 7: peripheral edge portion of the cap portion (3)
- 8: upright portion
- 9: position recessed from the peripheral edge of the body portion (2)by a 5% length (L) of a body length (D)
- D: horizontal length (diameter) of the body portion (2)
- H: height (depth) at the mid portion (6) of the cap portion (3)
- L: 5% length of the body length (D)
- R: radius of curvature of the surface of the peripheral edge portion(7) of the cap portion (3)
- α: upright angle
- β: angle at the position recessed from the peripheral edge of the body portion (2) by the 5% length (L) of the body length (D)

### Most Preferred Embodiment of the Invention

Hereafter, an explanation is given for an embodiment of the present invention based on the drawings.
Figs. 1 and 2 show the embodiment of the present invention. Fig. 1 is a partly broken perspective view of a tablet and Fig. 2 is a vertical sectional view of the tablet with the vicinity of its upright portion enlarged.

As shown in Fig. 1, a tablet 1 comprises a columnar body portion 2 of a predetermined thickness and cap portions 3 projecting form an upper and a lower surfaces of the body portion 2. This tablet 1 is formed by a generally used tabletting machine. Each of the upper and lower surfaces 4 of the body portion 2 has a peripheral edge portion provided with an annular land portion outside the cap portion 3.
This tablet 1 has a consistency so set that it promptly disintegrates in the oral cavity, by adjusting the tabletting pressure of the tabletting machine.

As shown in Figs. 1 and 2, the cap portion 3 has a mid portion 6 formed in a planar shape parallel to the upper and lower surfaces 4 of the body portion 2 and has a peripheral edge portion 7 the surface of which is curved so as to be smoothly continued with a surface of the mid portion 6. The mid portion 6 has a height, namely a depth of the cap portion 3, set to, for example, 0.3 to 1.5 mm.

The peripheral edge portion 7 has the surface which is formed arcuate in vertical section and has its radius of curvature so set that an upright portion 8 formed in the vicinity of an external end edge is provided with an upright angle (α) of 35 to 50 degrees, preferably 35 to 45 degrees with respect to the upper and lower surfaces 4 of the body portion 2. Further, the upright portion 8 has its surface connected to surface of the land portion 5 with a smooth curved surface.

The surface of the peripheral edge portion 7 is formed so that it has a large angle with respect to the upper and lower surfaces of the body portion 2 over a predetermined range. This body portion 2 has a peripheral edge portion recessed from a peripheral edge of the body portion 2 by a 5% length (L) of a horizontal length (D) (i.e. diameter) of the body portion 2 at a position 9 where the cap portion 3 has a surface provided with an angle (β) of at least 20 degrees with respect to the upper and lower surfaces 4. This angle (β) is preferably 20 to 90 degrees and more preferably 20 to 45 degrees.

Further, in this embodiment, the peripheral edge portion 7 has a surface formed in the shape of a convex curved surface so as to become arcuate in vertical section. This facilitate connecting the surface of the peripheral edge portion 7 to the mid portion 6 of the cap portion 3. However, according to the present invention, the peripheral edge portion 7 may have the surface formed linearly so as to be seen as inclined in vertical section. Moreover, although in this embodiment the mid portion is formed planar, according to the present invention, this mid portion may have a surface formed in a convex curved shape consisting of one or plural arcs in vertical section.
Furthermore, in this embodiment, the peripheral edge portion has the surface smoothly connected to the surface of the mid portion so that it is brought in contact therewith. However, according to the present invention, the peripheral edge portion does not necessarily have the surface brought into contact with the surface of the mid portion. In this case, there may be formed an angular portion at a portion for connecting the surface of the peripheral edge portion to the surface of the mid portion, but both of the surfaces are preferably connected to each other with a connecting curved surface.

### Example

The present invention will be explained by listing Examples and Comparison Examples in more detail, but it is not limited to those ones.

### Examples 1 to 7 and Comparison Examples 1 to 3

The preparation is as follows:

| | |
|---|---|
| HPC-L(binder) | 0.8 parts by wt |
| yellow ferric oxide | 0.1 parts by wt |

| (coloring agent) | |
|---|---|
| mannitol (excipient) | 76.9 parts by wt |
| corn starch (excipient) | 5.9 parts by wt |
| aspartame (sweetening agent) | 0.2 parts by wt |
| CEOLUS KG801 or KG802 | 10.0 parts by wt |

| (crystalline cellulose) | |
|---|---|
| (excipient) | |
| crospovidone (disintegrant) | 5.0 parts by wt |
| magnesium sterate (lubricant) | 1.0 parts by wt |
| voglibose (active ingredient) | 0.1 parts by wt |
| Total | 100.0 parts by wt |

The production method is as follows.
First, yellow ferric oxide was added to voglibose and HPC-L solution to prepare binding solution for the active ingredient. Next, mannitol, corn starch, aspartame, CEOLUS KG-801 OR KG-802 and crospovidone were granulated and then dried by using a fluidized-bed granulating and drying machine while spraying the active-ingredient binding solution. The thus resulting dry powder is subjected to a powder mill for uniformizing particles.
Subsequently, the particle-uniformized powder was introduced with corn starch and magnesium stearate into a tumbler mixing machine for mixing them. The mixture powder is tabletted by a rotary-type tabletting machine to obtain tablets of oral-disintegrable type.

In accordance with the above-mentioned preparation and production method, as for tables of one kind which weights 220 mg and those of the other kind which weights 150 mg, they were tabletted with their peripheral edge portions differentiated in section so that they have a hardness within a range (about of 40 to 50 N) and measurements were made for their abrasion ratios.
The hardness of the tablet was measured by Toyama hard meter.
Besides, as for the abrasion ratio, the tablets were treated by an abrasion-ratio measuring device which presumes the contact with other tablets and with the walls and then measurements were made for the thus treated tablets to find the occurrence ratio of the broken or cracked tablets. More specifically, this measuring device accommodated tablets in a hermetically sealed container and rotated the container for a predetermined period of time and thereafter the tablets were taken out of the container. The ratio of the weight of the tested total tablets to the weight of the pre-tested total tablets was measured and the results of this measurement are indicated in Comparison Table 1.

Apparently from this Comparison Table 1, when compared with Comparison Examples 1 to 3 each of which has a small upright angle, the respective Examples with each of their upright angles set to a large angle of about 41 degrees could reduce their abrasion ratios widely regardless of each of their sectional shapes.

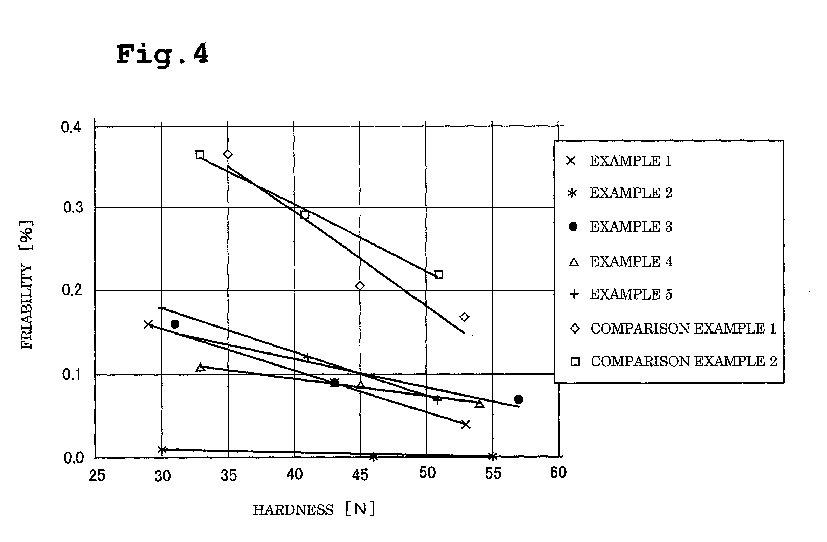

As regards the Examples 1 to 5 and the Comparison Examples 1 and 2, measurements were made for the ratios of the abrasions which occurred upon varying the hardness of every tablet by adjusting the tabletting pressure. The measurement results are shown in Comparison Graph of Fig. 4.
Apparently from this graph, as for the Comparison Examples 1 and 2 each having a small upright angle, the abrasion ratio is high. Besides, as the hardness of the tablet is progressively decreasing, the abrasion ratio becomes larger. On the contrary, as for the Examples 1 to 5 each of which has a large upright angle, in any case, the abrasion ratio was not only small but also could be inhibited from increasing so as to become lower even if each tablet decreases its hardness.

### Examples 8 to 10 and Comparison Examples 4 and 5

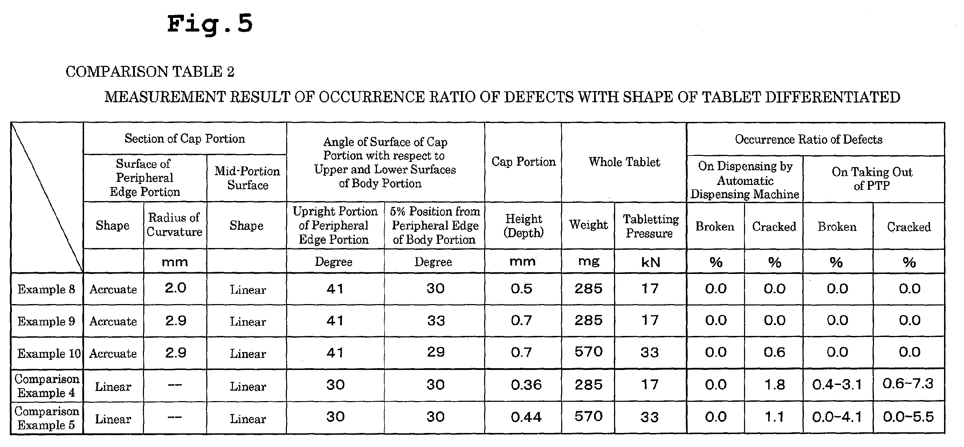

As for tables of one kind which weights 285 mg and those of the other kind which weights 570 mg, they were tabletted with 17N for the former and 33N for the latter while differentiating the shapes of the peripheral edge portions of the cap portions or the like in section, so that they have a hardness within a range. Then measurements were made for the ratio as to the breaking and cracking of the tablets which occurred upon dispensing by an automatic dispensing machine as well as upon taking them out of the PTP. The measurement results are indicated in Comparison Table 2 of Fig. 5.

As for the tablet dispensing machine, YS-TR-300FDS and CPX-45 manufactured by Yuyama Seisakusho Co., Ltd. or MAIN -TOPRA-384 manufactured by TOSHO Co., Ltd. was used.
Additionally, as to taking out of the PTP, measurements were made for the ratio as to the breaking and cracking which occurred in the tablets upon having actually taken out them in a medical organization or the like.

Apparently from this Comparison Table 2, when compared with Comparison Examples 4 and 5 each of which has a small upright angle, the respective Examples with each of their upright angles set to a large angle of about 41 degrees could reduce occurrence ratio of the defective products regardless of each of their sectional shapes on packing by the dispensing machine as well as on taking the tablet out of the PTP.

The tablets explained in the embodiment and Examples are illustrated for exemplifying purpose only so as to realize the technical idea of the present invention. Therefore, the dimension such as the length, width and thickness, and the shape of the tablet as well as the hardness thereof is not limited to those mentioned in the embodiment. Various modifications can be added thereto as far as they fall within a scope of claims of the present invention.
For example, in the embodiment, an explanation was made for a tablet circular in plan view, but the tablet of the present invention is not limited to that of a specific shape. It may be formed in any one of oval, elliptical and optional shapes. Needless to say, the pharmaceutical active ingredients to be included in the tablet are not limited to specific ingredients.

### Industrial Availability

The present invention is suitable particularly for the oral disintegrating tablet because the present invention can inhibit the occurrence of defective products without increasing the hardness of a tablet even if stress is applied to the tablet on transporting it or packing it by an automatic dispensing machine or when taking it out of the PTP and besides can be put into practice easily and inexpensively to result in facilitating the improvement of the productivity. But needless to say, it is applicable to the other tablets.

## Claims

1. A tablet comprising a body portion (2) of a predetermined thickness and a cap portion (3) projecting from at least one of an upper and a lower surfaces (4) of the body portion (2), wherein
the cap portion (3) has a peripheral edge portion (7) a surface of which is provided with an upright angle (α) of 35 to 50 degrees with respect to the upper and lower surfaces (4) of the body portion (2).

2. The tablet as set forth in claim 1, wherein
the cap portion (3) has the peripheral edge portion (7) formed in a shape of a convex curved surface which becomes arcuate in vertical section.

3. The tablet as set forth in claim 1 or 2, wherein
the cap portion (3) has its surface provided with an angle (β) of at least 20 degrees with respect to the upper and lower surfaces (4) at a position (9) of the peripheral edge portion (7) of the body portion (2), recessed by a 5% length (L) of a horizontal length (D) of the body portion (2), from a peripheral edge of the body portion (2).

4. The tablet as set forth in any one of claims 1 to 3, wherein
the cap portion (3) has a mid-portion (6) where at least most of its surface is formed planar parallel to the upper and lower surfaces (4).

5. The tablet as set forth in any one of claims 1 to 4, wherein
the cap portion (3) has the peripheral edge portion (7) whose surface is approximately smoothly connected to the surface at the mid portion (6).

6. The tablet as set forth in any one of claims 1 to 5, wherein
the cap portion (3) has the mid-portion (6) a height (H) of which is 0.3 to 1.5 mm.

7. The tablet as set forth in any one of claims 1 to 6, wherein
both of the body portion (2) and the cap portion (3) disintegrate in an oral cavity for a short time.
